# EUROPEAN PATENT APPLICATION

(11) **EP 4 710 874 A1**
(43) Date of publication of application: **18.03.2026**
(21) Application number: 24802837.5
(22) Date of filing: 29.04.2024
(51) Int. Cl.: A61B 17/34, A61B 17/221, A61F 2/01

(54) **FILTER SCREEN SHEATH**

(30) Priority: 06.05.2023 CN 202321073204 U
(71) Applicant: Shenzhen Intervenet Medical Technology Co., Ltd., Shenzhen, Guangdong 518042 (CN)
(72) Inventor: ZHANG, Wayne Wei, Shenzhen, Guangdong 518000 (CN); CHEN, Zhong, Shenzhen, Guangdong 518000 (CN); ZHANG, Xiong, Shenzhen, Guangdong 518000 (CN)
(74) Representative: Michalski Hüttermann & Partner Patentanwälte mbB
(86) International application number: PCT/CN2024/090581
(87) International publication number: WO 2024/230554

(57) **Abstract**

The present application relates to a filter screen sheath. The filter screen sheath includes an outer sheath tube and a filtering member capable of moving axially relative to the outer sheath tube; the filtering member includes a filter screen and a frame; the filter screen is provided with a proximal opening and a distal opening; the distal opening is communicated with a tube cavity of the outer sheath tube through the proximal opening; the frame includes a traction wire, a proximal supporting ring, and a distal supporting ring; the proximal supporting ring is connected to the proximal opening of the filter screen; the distal supporting ring is connected to the distal opening of the filter screen; and the traction wire is connected to the proximal supporting ring. The filter screen sheath of the present application allows a treatment instrument to pass through the filtering member, and the filtering member can capture an embolus in a blood vessel during treatment with the treatment instrument, thereby improving surgical safety.

## Description

### Technical Field

The embodiments relate to the field of medical instruments, and in particular, to a filter screen sheath.

### Background

In cardiovascular intervention surgery, a surgical instrument is often delivered to a target lesion for operation through a delivery sheath, such as balloon dilation and stent dilation for intravascular stenosis, stent reconstruction for aortic dissections and aortic aneurysms, and aortic valve replacement for valve diseases. All of the above surgeries may cause thrombi or calcified fragments in blood vessels to be detached during treatment. Without protection, the detached thrombi are flushed towards a distal end along with blood flow, causing distal vascular thrombosis. Vascular thrombosis in different parts of the body may lead to local pain, tissue necrosis, hemiplegia, dementia, and even a death risk to a patient.

In the existing art, a filter screen sheath is usually used at the distal end to intercept and capture detached emboli, to provide temporary protection for the cerebral blood vessels. However, the current filter screen sheath can only intercept and capture emboli, and cannot be compatible with treatment instruments at the same time.

### Summary

Based on this, it is necessary to provide a filter screen sheath that has a treatment function and a thrombus interception function in an interventional operation.

The embodiments provide a filter screen sheath, including an outer sheath tube and a filtering member capable of axially moving relative to the outer sheath tube.

The filtering member includes a filter screen and a frame. The filter screen is provided with a proximal opening and a distal opening. The distal opening is communicated with a tube cavity of the outer sheath tube through the proximal opening. The frame includes a traction wire, a proximal supporting ring, and a distal supporting ring. The proximal supporting ring is connected to the proximal opening of the filter screen. The distal supporting ring is connected to the distal opening of the filter screen. The traction wire is connected to the proximal supporting ring. Under the action of an external force, the traction wire drives the filtering member to axially move relative to the outer sheath tube.

In an embodiment, the distal end of the traction wire is fixedly connected to the proximal supporting ring, or the distal end portion of the traction wire is connected to the proximal supporting ring after being bent.

In an embodiment, the frame includes two traction wires.

The proximal supporting ring has a first opening. Two end portions of the first opening are respectively connected to the two traction wires.

In an embodiment, the frame further includes a connection wire. The connection wire is connected to the proximal supporting ring and the distal supporting ring.

In an embodiment, the frame includes two connection wires.

The proximal supporting ring has a second opening. Two end portions of the second opening are respectively connected to proximal ends of the two connection wires.

The distal supporting ring has a third opening. Two end portions of the third opening are respectively connected to distal ends of the two connection wires.

In an embodiment, a side hole is provided in a tube wall of the outer sheath tube. A proximal end of the traction wire is threaded from the side hole to an outer side of the outer sheath tube.

In an embodiment, the tube cavity of the outer sheath tube includes a first tube cavity and a second tube cavity that are parallel to each other.

The distance between a distal end surface of the second tube cavity and a distal end surface of the outer sheath tube in an axial direction of the outer sheath tube is D1. The maximum length of the filtering member in the axial direction of the outer sheath tube is L, and L<D1.

In an embodiment, the outer sheath tube includes an inner layer tube, a reinforcing tube, and an outer layer tube that are sleeved from inside to outside.

The distal end surface of the reinforcing tube is closer to the proximal end of the outer sheath tube than the distal end surface of the inner layer tube and the distal end surface of the outer layer tube, to enclose the distal end surface of the reinforcing tube, a peripheral surface of the inner layer tube, and an inner wall surface of the outer layer tube to form an annular receiving space. The annular receiving space faces the distal opening.

The side hole is communicated with the annular receiving space.

In an embodiment, an annular groove is provided in an inner wall surface of the outer sheath tube. The annular groove is coaxial with the outer sheath tube. The annular groove is configured to provide clearance for the proximal supporting ring.

The distance between the annular groove and the distal end surface of the outer sheath tube in the axial direction of the outer sheath tube is D2. The shortest axial distance between the proximal supporting ring and the distal supporting ring is 1, and 1>D2.

In an embodiment, a stopper is arranged on an inner wall surface of the outer sheath tube. The stopper is close to a distal end of the outer sheath tube. The height of the stopper is less than or equal to the wall thickness of the proximal supporting ring.

The filter screen of the present application has the proximal opening and the distal opening. When the filtering member is in a released state, the distal opening is communicated with the tube cavity of the outer sheath tube through the proximal opening, thereby establishing a delivery channel, so that a treatment instrument can pass through the delivery channel. The proximal supporting ring of the filtering member can support the proximal end of the filtering member, thereby avoiding interference with the passing of the treatment instrument due to collapse of the proximal end of the filtering member. After the treatment instrument passes through the filtering member, when a site of lesion in a blood vessel is treated, an air embolus carried by the treatment instrument and a thrombus detached from the blood vessel can be captured by the filtering member, thereby improving surgical safety.

### Brief Description of the Drawings

FIG. 1 is a schematic diagram of a filter screen sheath in Embodiment 1;
FIG. 2 is a schematic diagram of a filtering member in Embodiment 1;
FIG. 3 is a schematic diagram of a frame in Embodiment 1;
FIG. 4 is a schematic diagram of a frame in other embodiments (the frame is provided with a traction wire and two connection wires);
FIG. 5 is a schematic diagram of a frame in other embodiments (the frame is only provided with a traction wire and a connection wire);
FIG. 6 is a schematic diagram of the assembly of a filter screen sheath and a treatment instrument in Embodiment 1 (profile treatment is performed on the outer sheath tube);
FIG. 7 is a longitudinal sectional view of an outer sheath tube in Embodiment 1;
FIG. 8 is an enlarged view of part A in FIG. 7;
FIG. 9 is a longitudinal sectional view of an outer sheath tube in Embodiment 2;
FIG. 10 is a sectional view taken along line B in FIG. 9;
FIG. 11 is a longitudinal sectional view of an outer sheath tube in Embodiment 3;
FIG. 12 is a longitudinal sectional view of a filter screen sheath in Embodiment 3;
FIG. 13 is a longitudinal sectional view of a filter screen sheath in other embodiments;
FIG. 14 is a longitudinal sectional view of an outer sheath tube in Embodiment 4; and
FIG. 15 is an enlarged view of part C in FIG. 14.

### Detailed Description of the Invention

In order to make the objectives, technical solutions, and advantages of the embodiments clearer, the following is a further detailed explanation of the embodiments in conjunction with the accompanying drawings and embodiments. It should be understood that the specific embodiments described here are only intended to explain the embodiments and are not intended to limit the embodiments.

Unless otherwise defined, all technical and scientific terms used herein have the same meaning as those commonly understood by a person skilled in the art to which the present disclosure belongs. Terms used in the specification of the present disclosure herein are merely intended to describe objectives of the specific embodiments, but are not intended to limit the embodiments. The term "and/or" used herein includes any and all combinations of one or more related listed items.
In order to more clearly describe structures of the embodiments, "distal end" and "proximal end" are used as localizers. The localizers are conventional terms in the field of interventional medical instruments. A "distal end" is defined as an end that is far away from an operator in a surgical process, and a "proximal end" is defined as an end that is close to the operator in the surgical process.

### Embodiment 1

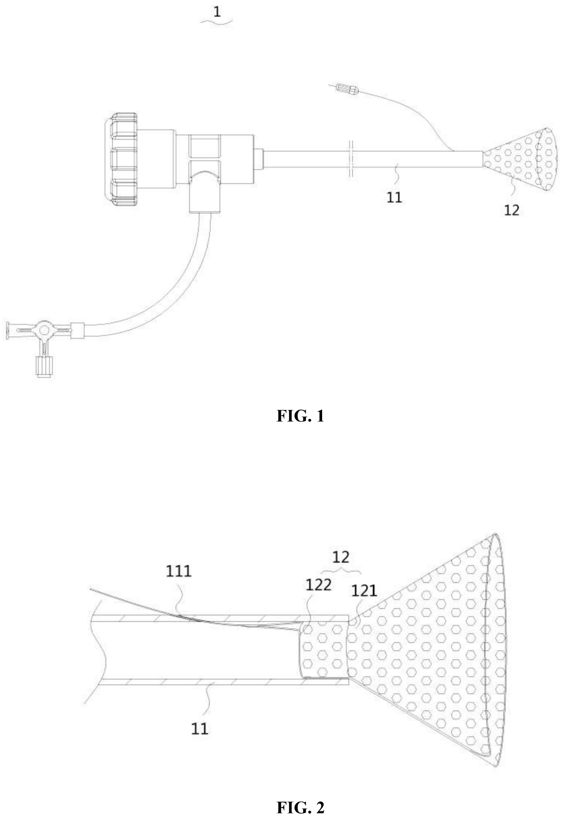

As shown in FIG. 1, a filter screen sheath 1 of this embodiment includes an outer sheath tube 11 and a filtering member 12 capable of moving axially relative to the outer sheath tube 11.

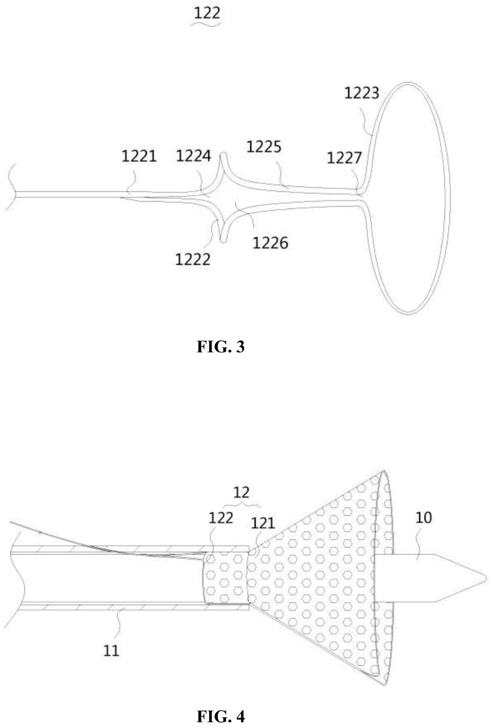

As shown in FIG. 2, the filtering member 12 includes a filter screen 121 and a frame 122. The filter screen 121 has a proximal opening and a distal opening. The distal opening is communicated with a tube cavity of the outer sheath tube 11 through the proximal opening. As shown in FIG. 3, the frame 122 includes a traction wire 1221, a proximal supporting ring 1222, and a distal supporting ring 1223. The proximal supporting ring 1222 is connected to the proximal opening of the filter screen 121. The distal supporting ring 1223 is connected to the distal opening of the filter screen 121. The traction wire 1221 is connected to the proximal supporting ring 1222. Under the action of an external force, the traction wire 1221 drives the filtering member 12 to move axially relative to the outer sheath tube 11.

The filter screen 121 of this embodiment has the proximal opening and the distal opening. As shown in FIG. 4, when the filtering member 12 is in a released state, the distal opening is communicated with the tube cavity of the outer sheath tube 11 through the proximal opening, thereby establishing a delivery channel, so that a treatment instrument 10 can pass through the delivery channel. The proximal supporting ring 1222 of the filtering member 12 can support the proximal end of the filtering member 12, thereby avoiding interference with the passing of the treatment instrument 10 due to collapse of the proximal end of the filtering member 12. After the treatment instrument 10 passes through the filtering member 12, when a site of lesion in a blood vessel is treated, an air embolus carried by the treatment instrument 10 and a thrombus detached from the blood vessel can be captured by the filtering member 12, thereby improving surgical safety.

The material of the filter screen 121 is selected from a metal woven screen, a polymer material fiber woven or spun screen, a polymer mixed metal material woven or spun screen, or a porous polymer film. The pore diameter of the filter screen is 0.1 to 0.5 mm.

In this embodiment, referring to FIG. 3, the filter screen sheath 1 includes two traction wires 1221. The proximal supporting ring 1222 has a first opening 1224, and the two end portions of the first opening 1224 are respectively connected to the two traction wires 1221.

The frame 122 includes two connection wires 1225. The proximal supporting ring 1222 has a second opening 1226. Two end portions of the second opening 1226 are respectively connected to proximal ends of the two connection wires 1225. The distal supporting ring 1223 has a third opening 1227. Two end portions of the third opening 1227 are respectively connected to distal ends of the two connection wires 1225.

In this embodiment, the two traction wires 1221, the proximal supporting ring 1222, the two connection wires 1225, and the distal supporting ring 1223 are formed by bending a single metal wire multiple times. After the metal wire is bent to form the distal supporting ring 1223 with the third opening 1227, two ends of the distal supporting ring 1223 are respectively bent to form the two connection wires 1225. The two connection wires 1225 are bent respectively to form the proximal supporting ring 1222 with the first opening 1224 and the second opening 1226. The proximal supporting ring 1222 is bent at the first opening 1224 to form the two traction wires 1221.

In this embodiment, the two traction wires 1221 have different lengths. A proximal end of the shorter traction wire 1221 is fixedly connected to the longer traction wire 1221, to reduce the space occupied by the traction wire 1221 in the outer sheath tube 11 or reduce a pore diameter required by the traction wires 1221 to pass through a tube wall of the outer sheath tube 11. It can be understood that shorter traction wires 1221 is more advantageous to reduce the space occupied by the two traction wires 1221. Therefore, based on meeting shape and strength requirements for forming the proximal supporting ring 1222, the length of one of the traction wires 1221 can be shortened as much as possible.

Under the action of an external force, the traction wires 1221 drive the filtering member 12 to move axially relative to the outer sheath tube 11. For example, when a doctor applies tension to the traction wires 1221, the frame 122 deforms to bundle the entire filtering member 12 within the outer sheath tube 11. The frame 122 of this embodiment is formed by bending a metal wire multiple times, so that the procedures are simple, and the overall connection strength is high.

In order to achieve pushing and bundling of the filtering member 12, the traction wires 1221 need to have bending resistance, and the proximal supporting ring 1222, the connection wires 1225, and the distal supporting ring 1223 need to have deformability. For example, the diameter of each traction wire 1221 is greater than the diameter of each of metal wire sections that form the proximal supporting ring 1222, the connection wires 1225, and the distal supporting ring 1223. In this embodiment, the frame 122 is overall made of a nickel titanium wire. The diameter of each traction wire 1221 is 0.3 mm to 0.4 mm. The diameter of each of nickel titanium wire sections that form the proximal supporting ring 1222, the connection wires 1225, and the distal supporting ring 1223 is 0.08 mm to 0.1 mm. In other embodiments, each traction wire 1221 is made of a stainless steel wire, and the proximal supporting ring 1222, the connection wires 1225, and the distal supporting ring 1223 are made of nickel titanium wires.

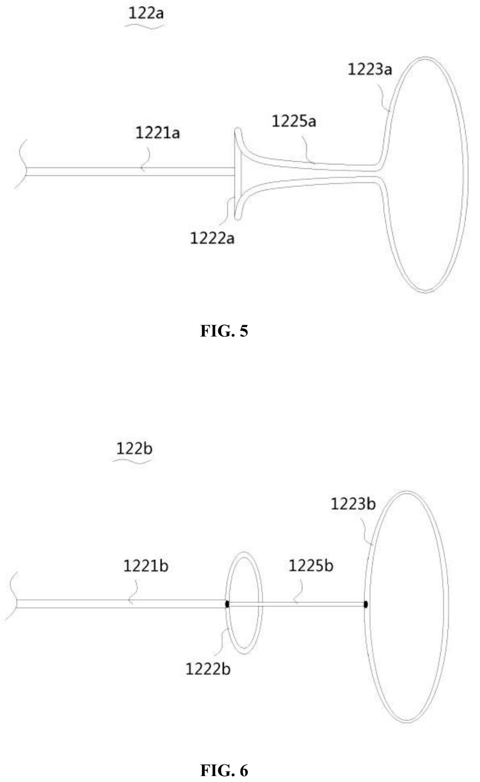

In other embodiments, distal ends of the traction wires are fixedly connected to the proximal supporting ring. Alternatively, distal ends of the traction wires are connected to the proximal supporting ring after being bent. For example, the distal ends are connected to the supporting ring after being bent into rings. Similarly, the connection wires may be fixedly connected to the proximal supporting ring and distal supporting rings, or end portions of the connection wires may be bent into circles and then connected to the proximal supporting ring and the distal supporting ring. As shown in FIG. 5, the frame 122a includes a traction wire 1221a and two connection wires 1225a. A distal end of the traction wire 1221a is connected to a proximal supporting ring 1222a, and the two connection wires 1225a are respectively connected to openings of the proximal supporting ring 1222a and the distal supporting ring 1223a. As shown in FIG. 6, the frame 122b includes a traction wire 1221b and a connection wire 1225b. The traction wire 1221b is directly connected to a proximal supporting ring 1222b, and the connection wire 1225b is directly connected to the proximal supporting ring 1222b and a distal supporting ring 1223b.

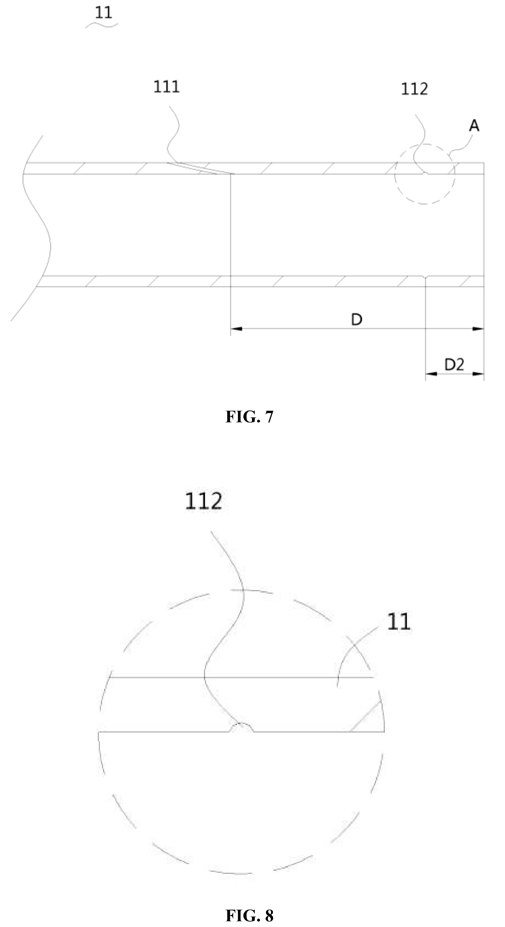

In this embodiment, as shown in FIG. 7, a side hole 111 is provided in a tube wall of the outer sheath tube 11. A proximal end of the traction wire 1221 is threaded from the side hole 111 to an outer side of the outer sheath tube 11. A distance D between the most distal end of the side hole 111 and the distal end surface of the outer sheath tube 11 should be greater than the length of the filtering member 12 in the axial direction of the outer sheath tube 11, so that the filtering member 12 can be completely bundled inside the outer sheath tube 11. By the arrangement of the side hole 111, entanglement between the traction wire 1221 and the treatment instrument 10 that needs to pass through the tube cavity of the outer sheath tube 11 can be avoided.

In other embodiments, the traction wire is arranged inside the tube cavity of the outer sheath tube.

In this embodiment, as shown in FIG. 7 and FIG. 8, an annular groove 112 is provided in an inner wall surface of the outer sheath tube 11. The annular groove 112 is coaxial with the outer sheath tube 11. The annular groove 112 is configured to provide clearance for the proximal supporting ring 1222. When the filtering member 12 is entirely bundled inside the outer sheath tube 11, the proximal supporting ring 1222 resists against an inner wall of the tube cavity of the outer sheath tube 11. When the filtering member 12 is pushed by an external force to the proximal supporting ring 1222 to directly face the annular groove 112, the proximal supporting ring 1222 is clamped into the annular groove 112 by its own elasticity, thereby reducing an area occupied by the proximal supporting ring 1222 on a cross section of the outer sheath tube 11, and then allowing a treatment instrument 10 with a larger outer diameter to pass through the filtering member 12.

The depth of the annular groove 112 is less than or equal to the diameter of the metal wire for making the proximal supporting ring 1222. This is because if the annular groove 112 is too deep, it is not conducive for the proximal supporting ring 1222 to be separated from the groove when the filtering member 12 is withdrawn. For example, the outer sheath tube 11 includes an inner layer tube, an outer layer tube, and a reinforcing tube sandwiched between the inner layer tube and the outer layer tube. The reinforcing tube is of a helical tube structure. The helical tube structure includes a plurality of helical coils connected in sequence. During the making of the helical tube structure, the diameter of one or several adjacent spiral coils is increased to form the annular groove 112 in the inner wall of the spiral tube structure. The inner layer tube and the outer layer tube are both made of polymer materials and are respectively attached to inner and outer walls of the reinforcing tube, to form the outer sheath tube 11 with the annular groove 112 in this embodiment.

The distance between the annular groove 112 and the distal end surface of the outer sheath tube 11 in the axial direction of the outer sheath tube 11 is D2. The shortest axial distance between the proximal supporting ring 1222 and the distal supporting ring 1223 is 1, and 1>D2. When the proximal supporting ring 1222 is clamped into the annular groove 112, the distal supporting ring 1223 is completely exposed out of the outer sheath tube 11, and the distal supporting ring 1223 abuts against a vascular wall, to avoid blood from flowing directly through a gap between the filtering member 12 and the vascular wall. Therefore, the annular groove 112 not only is configured to provide clearance for the proximal supporting ring 1222, but also serves as a positioning member of the filtering member 12. During the release of the filtering member 12, the traction wire 1221 remains stationary, and the outer sheath tube 11 is withdrawn. When resistance increases significantly (the proximal supporting ring 1222 falls into the annular groove 112), the withdrawal of the outer sheath tube 11 is stopped, so that the filtering member 12 is in a preset release position.

### Embodiment 2

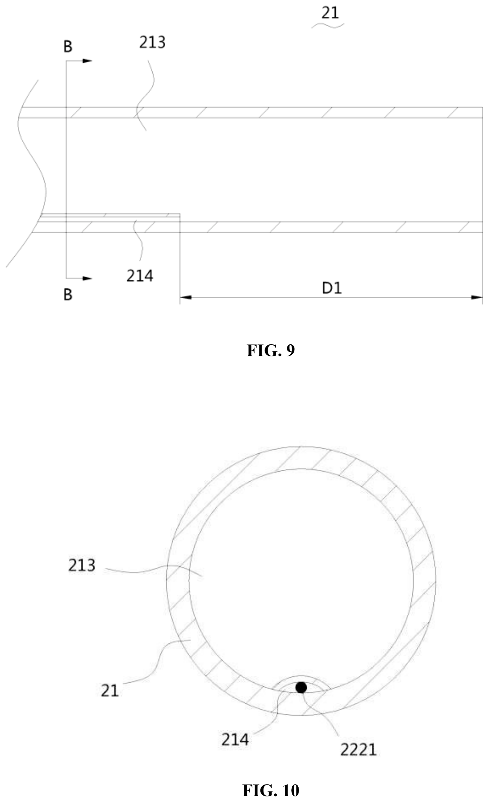

The filter screen sheath of this embodiment is basically the same as the filter screen sheath of Embodiment 1, and only an outer sheath tube 21 is different. As shown in FIG. 9 and FIG. 10, a tube cavity of the outer sheath tube 21 of this embodiment includes a first tube cavity 213 and a second tube cavity 214 that are parallel to each other. The first tube cavity 213 is configured to allow a treatment instrument to pass through, and the second tube cavity 214 is configured to accommodate the traction wire 2221, to avoid entanglement between the traction wire 2221 and the treatment instrument that needs to pass through the outer sheath tube 21.

In order to completely bundle the filter screen sheath into the outer sheath tube 21, thr distance between a distal end surface of the second tube cavity 214 and a distal end surface of the outer sheath tube 21 in the axial direction of the outer sheath tube 21 is D1. A maximum length of the filtering member in the axial direction of the outer sheath tube is L, and L<D1.

### Embodiment 3

The filter screen sheath of this embodiment is basically the same as the filter screen sheath of Embodiment 1, and only an outer sheath tube 31 is different.

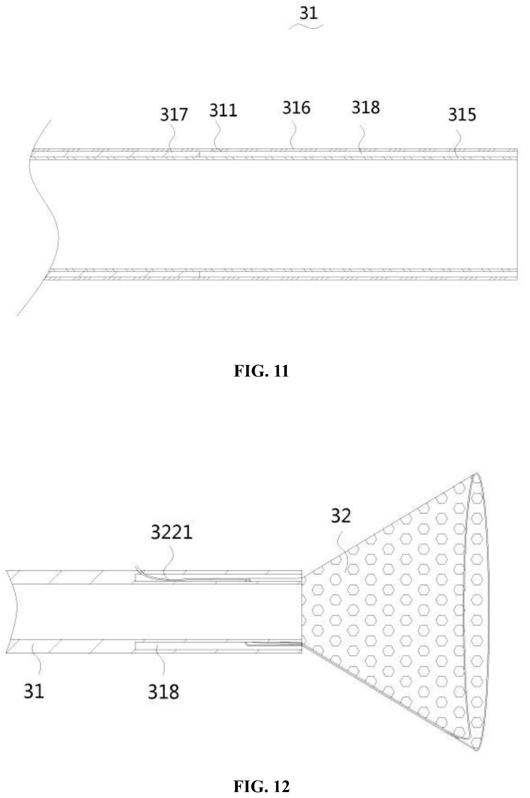

As shown in FIG. 11, the outer sheath tube 31 includes an inner layer tube 315, a reinforcing tube 317, and an outer layer tube 316 that are sleeved from inside to outside. The distal end surface of the reinforcing tube 317 is closer to the proximal end than the distal end surface of the inner layer tube 315 and the distal end surface of the outer layer tube 316, to enclose the distal end surface of the reinforcing tube 317, a peripheral surface of the inner layer tube 315, and an inner wall surface of the outer layer tube 316 to form an annular receiving space 318. The annular receiving space 318 faces the distal opening. The side hole 311 is communicated with the annular receiving space 318.

As shown in FIG. 12, the proximal end of the filtering member 32 is limited within the annular receiving space 318, and the proximal end of the traction wire 3221 extends from the annular receiving space 318 to an outer side of the outer sheath tube 31 through the side hole 311. The traction wire 3221 can be pulled to pull the entire filtering member 32 into the annular receiving space 318. By modifying the structure of the outer sheath tube 31 itself, occupation of the cross-sectional area of the tube cavity of the outer sheath tube 31 by the proximal supporting ring is reduced.

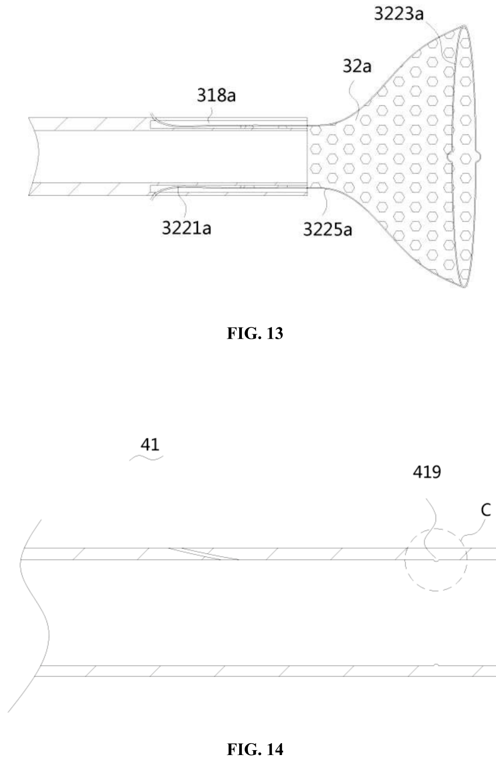

In other embodiments, as shown in FIG. 13, the frame only includes a distal supporting ring 3223a, two connection wires 3225a, and two traction wires 3221a. Since the proximal end of the filtering member 32a is limited within the annular receiving space 318a, no proximal supporting ring is required. The two traction wires 3221a are directly connected to the connection wires 3225a.

### Embodiment 4

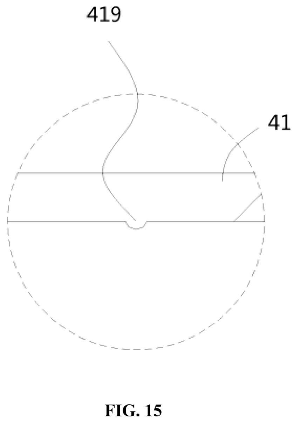

The filter screen sheath of this embodiment is basically the same as the filter screen sheath of Embodiment 1, and only the outer sheath tube 41 is different. As shown in FIG. 14 and FIG. 15, a stopper 419 is arranged on an inner wall surface of the outer sheath tube 41 of this embodiment. The stopper 419 is close to a distal end of the outer sheath tube 41. The height of the stopper 419 is less than or equal to the wire diameter of the proximal supporting ring. The stopper 419 is mainly configured to stop the proximal supporting ring from continuing to move towards the distal end, thereby avoiding such the phenomenon that withdrawal is difficult when the proximal end of the filtering member is detached from the outer sheath tube 41. The height of the stopper 419 is less than or equal to the wire diameter of the proximal supporting ring, which can prevent the stopper 419 from occupying an area of a cross section of the outer sheath tube 41.

The stopper 419 may be a continuous circle of protrusion or may be a plurality of independent protrusions. The stopper 419 may be arranged close to the distal end of the outer sheath tube 41 or may be flush with the distal end surface of the outer sheath tube 41.

The various technical features in the foregoing embodiments may be randomly combined. For concise description, not all possible combinations of the various technical features in the above embodiments are described. However, provided that combinations of these technical features do not conflict with each other, the combinations of the various technical features are considered as falling within the scope of this specification.

The foregoing embodiments merely express several implementations of the present disclosure. The descriptions thereof are relatively specific and detailed, but are not understood as limitations on the scope of the present disclosure. A person of ordinary skill in the art can also make several transformations and improvements without departing from the idea of the present disclosure. These transformations and improvements fall within the protection scope of the present disclosure. Therefore, the protection scope of the patent of present disclosure shall be subject to the appended claims.

## Claims

1. A filter screen sheath, comprising an outer sheath tube and a filtering member capable of moving axially relative to the outer sheath tube,
wherein the filtering member comprises a filter screen and a frame; the filter screen is provided with a proximal opening and a distal opening; the distal opening is communicated with a tube cavity of the outer sheath tube through the proximal opening; the frame comprises a traction wire, a proximal supporting ring, and a distal supporting ring; the proximal supporting ring is connected to the proximal opening of the filter screen; the distal supporting ring is connected to the distal opening of the filter screen; the traction wire is connected to the proximal supporting ring; and under the action of an external force, the traction wire drives the filtering member to axially move relative to the outer sheath tube.

2. The filter screen sheath according to claim 1, wherein
a distal end of the traction wire is fixedly connected to the proximal supporting ring, or a distal end portion of the traction wire is connected to the proximal supporting ring after being bent.

3. The filter screen sheath according to claim 1, wherein
the frame comprises two traction wires;
the proximal supporting ring has a first opening; and two end portions of the first opening are respectively connected to the two traction wires.

4. The filter screen sheath according to claim 1, wherein
the frame further comprises a connection wire; and the connection wire is connected to the proximal supporting ring and the distal supporting ring.

5. The filter screen sheath according to claim 4, wherein
the frame comprises two connection wires;
the proximal supporting ring has a second opening; and two end portions of the second opening are respectively connected to proximal ends of the two connection wires;
the distal supporting ring has a third opening; and two end portions of the third opening are respectively connected to distal ends of the two connection wires.

6. The filter screen sheath according to claim 1, wherein
a side hole is provided in a tube wall of the outer sheath tube; and a proximal end of the traction wire is threaded from the side hole to an outer side of the outer sheath tube.

7. The filter screen sheath according to claim 1, wherein
the tube cavity of the outer sheath tube comprises a first tube cavity and a second tube cavity that are parallel to each other;
the distance between a distal end surface of the second tube cavity and a distal end surface of the outer sheath tube in an axial direction of the outer sheath tube is D1; and the maximum length of the filtering member in the axial direction of the outer sheath tube is L, and L<D 1.

8. The filter screen sheath according to claim 7, wherein
the outer sheath tube comprises an inner layer tube, a reinforcing tube, and an outer layer tube that are sleeved from inside to outside;
the distal end surface of the reinforcing tube is closer to the proximal end of the outer sheath tube than the distal end surface of the inner layer tube and the distal end surface of the outer layer tube, to enclose the distal end surface of the reinforcing tube, a peripheral surface of the inner layer tube, and an inner wall surface of the outer layer tube to form an annular receiving space; the annular receiving space faces the distal opening; and
the side hole is communicated with the annular receiving space.

9. The filter screen sheath according to any one of claim 6 or 7, wherein
an annular groove is provided in an inner wall surface of the outer sheath tube; the annular groove is coaxial with the outer sheath tube; the annular groove is configured to provide clearance for the proximal supporting ring;
the distance between the annular groove and the distal end surface of the outer sheath tube in the axial direction of the outer sheath tube is D2; and the shortest axial distance between the proximal supporting ring and the distal supporting ring is 1, and 1>D2.

10. The filter screen sheath according to claim 1, wherein
a stopper is arranged on an inner wall surface of the outer sheath tube; the stopper is close to a distal end of the outer sheath tube; and the height of the stopper is less than or equal to the wire diameter of the proximal supporting ring.
